# FASCICULE DE BREVET EUROPEEN

(11) **EP 2 119 474 B1**
(45) Date de publication et mention de la délivrance du brevet: **06.04.2016**
(21) Numéro de dépôt: 09159081.0
(22) Date de dépôt: 29.04.2009
(51) Int. Cl.: A61N 1/375, H04R 25/00, A61N 1/05, A61N 1/372, A61N 1/36

(54) **Dispositif implantable sous-cutané**
Subkutan implantierbare Vorrichtung
Subcutaneously implantable device

(30) Priorité: 15.05.2008 FR 0853147
(43) Date de publication de la demande: 18.11.2009
(73) Titulaire: Neurelec, 06220 Vallauris (FR)
(72) Inventeur: Charvin, Guy, 06600 ANTIBES (FR)
(74) Mandataire: Nielsen, Hans Jørgen Vind

(56) Documents cités:
- WO-A-03/092326
- WO-A-2005/110540
- GB-A- 2 134 335
- US-A1- 2005 171 579
- US-A1- 2006 184 212
- US-A1- 2007 255 349
- US-B1- 6 308 101
- US-B1- 7 200 504

## Description

La présente invention à pour objet un dispositif implantable sous-cutané apte à être placé en tout endroit d'un corps vivant, directement sous la peau de celui-ci pour émettre et/ou recevoir des données en direction ou en provenance de l'extérieur dudit corps, tel qu'au moins d'un autre dispositif disposé extérieurement audit corps.

Une des applications principales de cette invention est de réaliser des prothèses auditives destinées à corriger les surdités profondes d'un être humain et qui comportent pour cela des électrodes soit implantées dans la cochlée, soit extracochléaires et disposées sur la fenêtre ronde ou ovale de celle-ci, et destinées à transmettre des signaux électriques aux fibres du nerf auditif qui se trouve situé dans celle-ci.

De tels implants cochléaires sont connus et remplacent ainsi des cochlées déficientes, stimulant directement le nerf auditif en fonction des sons captés par un microphone externe : celui-ci est situé en général dans un boîtier comprenant en outre un microprocesseur et un support de type contour d'oreille pour le placer discrètement en arrière et autour de celle-ci.

Les sons captés ainsi par le microphone sont numérisés et traités dans ce boîtier externe qui adresse grâce à un jeu d'antennes (associées au moins pour l'une à ce boîtier externe, et pour une autre, sous-cutanée, à l'implant), des signaux d'informations correspondant à ces sons, à l'implant disposé sous la peau.

L'implant comprend en général un petit boîtier constitué de titane et de silicone pour être biocompatible et placé chirurgicalement sous la peau : il comporte des composants électroniques et reçoit les signaux d'informations de l'antenne extérieure grâce à sa propre antenne et pour les dispositifs à électrodes intracochléaires les répartit vers et sur ces électrodes qui ont été placées dans la cochlée : comme sur le clavier d'un piano chaque électrode correspond à une bande de fréquences de signal sonore et les terminaisons du nerf auditif en contact avec les électrodes vont transmettre les impulsions électriques jusqu'au cerveau qui les interprète comme des sons.

Par ailleurs, comme un tel implant nécessite un minimum d'énergie, celle-ci lui est fournie grâce à un couplage électromagnétique de bobines situées face à face dans les dispositifs implantable et externe respectivement et maintenus centrés l'une par rapport à l'autre grâce à un jeu d'aimants (solidaires respectivement des dispositifs implantable et externe) : leur rendement est certes faible puisqu'il n'y pas d'entrefer mais est suffisant pour le peu de quantité d'énergie nécessaire, et à condition de choisir et de disposer les divers composants judicieusement. Certains dispositifs implantables comportent également des batteries rechargeables.

D'autres applications sont bien sûr possibles chaque fois qu'il est nécessaire ou utile de pouvoir recueillir des données sur un organe en utilisant des électrodes dites de recueils physiologiques, ou au contraire d'envoyer des informations à cet organe (comme pour la cochlée) depuis l'extérieur sans perforation permanente de la peau.

De tels dispositifs sont connus et développés par différents fabricants qui ont déposés diverses demandes de brevet tel que :
- le brevet EP 999 874 de la société ADVANCED BIONICS CORPORATION et ALFRED E. MANN FOUNDATION FOR SCIENTIFIC RESEARCH et intitulé "Dispositif implantable à configuration améliorée d'alimentation et de recharge de batterie" déposé le 31 juillet 1998,
- la demande de brevet EP 1 166 820 déposée par la société Medtronic, Inc. intitulée "dispositif médical implantable avec une bobine externe de rechargement électrique" déposée le 19 juin 2000,
- ou encore le brevet US 6,246,911 de Monsieur Peter Seligman déposé le 1er juillet 1999.

Tous ces fabricants ont du étudier et faire des compromis dans la conception de leurs dispositifs pour satisfaire simultanément des contraintes contradictoires telles que : la nécessité de biocompatibilité des implants, la mise en oeuvre la plus simple possible pour le chirurgien, l'encombrement minimum, un rendement suffisant à la fois dans la transmission des données et dans le rechargement électrique de l'implant (rendement faible en effet du fait de la proximité des antennes de réception/émission avec le boîtier en titane, et du mauvais couplage électromagnétique des bobines pour le transfert d'énergie), ainsi que la présence d'aimants qui de plus perturbent les imageries de type IRM, et que l'on doit pouvoir faire sur le corps du patient concerné.

Il est donc nécessaire entre autres de diminuer au maximum les dimensions du dispositif implantable tant en épaisseur qu'en surface tout en ayant la possibilité de pouvoir en ôter l'aimant de centrage sans avoir à enlever l'implant, afin de pouvoir faire de tels imageries.

Pour cela tous les fabricants à ce jour ont choisi comme compromis de placer la partie antenne et bobine de l'implant ainsi que son aimant dans une protection de type enveloppe en silicone et de disposer cet ensemble sur le côté d'un boîtier en titane contenant la partie électronique et la connexion aux électrodes cochléaires, comme décrit dans la demande de brevet WO 03/092326 de la société COCHLEAR LTD ; l'aimant, qui est maintenu en place par l'enveloppe silicone disposant d'une fente, peut être enlevé par une petite incision une fois l'implant en place, et l'antenne et la bobine, décalées du boîtier en titane, sont moins perturbées par celui-ci et permettent un rendement satisfaisant.

L'antenne et la bobine peuvent être soit séparées, soit constituées par un même composant qui assure alors à la fois la transmission des données et le couplage électromagnétique de transfert d'énergie ; dans la présente description, on désignera comme bobine tout composant qui permet soit d'assurer uniquement le couplage électromagnétique de transfert d'énergie, soit d'assurer à la fois, et même éventuellement en même temps, cette fonction ainsi que celle d'une antenne de transmission de données.

La présente invention permet d'aller plus loin dans la recherche de la diminution de l'encombrement du dispositif implantable avec de plus une plus grande résistance de celui-ci, tout en ayant de bonnes et suffisantes caractéristiques et qualité de transmission et de recharge électrique et tout en permettant l'extractibilité de l'aimant implanté.

Pour cela un dispositif implantable dans un corps vivant, sous-cutané et apte à émettre et/ou recevoir des données en direction ou en provenance de l'extérieur dudit corps vivant et à recevoir de l'énergie par couplage électromagnétique avec une bobine d'un dispositif externe, comporte, comme dans les dispositifs connus à ce jour, tel que celui décrit dans la demande de brevet WO 03/092326, au moins une bobine, un boîtier étanche comprenant des moyens électroniques au moins de stimulation et/ou de recueil et un aimant apte à maintenir et à centrer la bobine dudit autre dispositif externe avec celle du dispositif implantable et il est tel que, suivant l'invention :
- ledit boîtier comporte une couronne creuse, formant une gorge annulaire en forme de U, en matériau biocompatible et perméable aux ondes électromagnétiques, contenant au moins lesdits moyens électroniques et ladite bobine, et un fond en matériau biocompatible fermant de manière étanche l'ouverture de la partie creuse de la couronne,
- ledit aimant est de dimensions compatibles avec le logement central formé par la paroi dite intérieure de la couronne et ouvert au moins d'un côté, et dans lequel il est placé.

Dans des modes préférentiels de réalisation l'aimant est placé dans le logement central d'une manière réversible et extractible, et le matériau de la couronne creuse est de la céramique alors que le matériau du fond est du titane.

Dans l'application principale de l'invention, le dispositif comprend au moins une électrode filaire extérieure, qui peut donc être une électrode cochléaire, comprenant en fait plusieurs électrodes et dont l'extrémité distale est apte à être logée sur, contre ou dans tout organe vers lequel et/ou duquel lesdites données sont adressées et/ou recueillies par les moyens électroniques du dispositif implantable : cette électrode filaire et qui est connectée, par son extrémité proximale, préférentiellement à la périphérie radiale de la couronne creuse du boîtier de ce dispositif implantable, et en fait de préférence en plusieurs points de connections correspondant chacun à une électrode de l'électrode filaire.

Le résultat est un nouveau dispositif implantable sous-cutané très compact qui peut avoir une épaisseur de moins de 5 mm pour 30 mm de diamètre externe ce qui limite à la fois sa surface extérieure mais également les dimensions de la partie du dispositif externe qui lui fait face : sa mise en place est ainsi facilitée, réduisant également les traumatismes de décollement de la peau, et l'implant fait moins d'ombres lors d'imagerie de type IRM ou radio ; le dispositif implantable permet également d'avoir suivant l'invention l'aimant de l'implant plus proche de la peau, assurant une plus grande attraction avec l'aimant extérieur, ce qui permet de diminuer la force de ces aimants et donc leurs dimensions et ainsi globalement de réduire la taille de l'ensemble des dispositifs.

De plus la réalisation du boîtier en céramique offre une plus grande résistance aux chocs à la fois par le matériau et par la forme de la partie creuse en couronne formant le logement de la partie électronique et de la bobine et qui constitue une arche en U plus courte que celle d'un boîtier en forme de disque.

Certes la présence du fond en titane, même s'il ne constitue pas une cage fermée de Faraday comme les boîtes entièrement en titane, dégrade les transmissions de signaux mais les dispositifs électroniques que tout homme de métier peut réaliser avec ses connaissances actuelles sont suffisamment performant pour compenser.

La description suivante se réfère aux dessins annexés qui représentent sans aucun caractère limitatif des exemples de réalisations du dispositif selon l'invention.
La figure 1 est une vue générale d'un exemple de prothèse auditive à électrodes intracochléaires et pouvant utiliser un dispositif suivant l'invention.
Les figures 2A et 2B sont les vues respectivement de dessus et de profil d'un dispositif implantable connu dans le domaine de la prothèse auditive.
La figure 3 est une vue perspective simplifié d'un dispositif suivant l'invention représenté à titre d'exemple dans le domaine des prothèses auditives cochléaire à électrode filaire.
Les figures 4A, 4B et 4C sont des vues en coupe de trois modes de réalisation du boîtier étanche d'un dispositif implantable suivant l'invention et tel que représenté sur la figure 3.

Toute la description ci-après des figures n'est qu'un exemple de réalisation qui se réfère à l'application principale de l'invention qui est de réaliser des prothèses auditives comprenant un dispositif implantable sous-cutané ; mais le dispositif suivant l'invention pourrait être utilisé dans d'autres applications, chaque fois qu'il est nécessaire ou utile de pouvoir recueillir des données sur un organe en utilisant par exemple des électrodes dites de recueils physiologiques ou au contraire d'envoyer des informations à cet organe (comme pour la cochlée prise à titre d'exemple ci-après), depuis l'extérieur du corps vivant considéré sans perforation permanente de la peau.

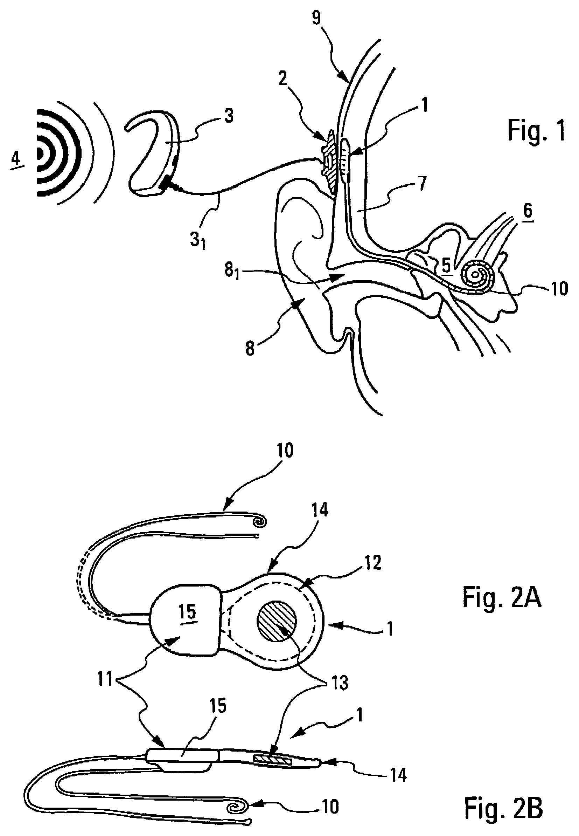

Dans le présent exemple de prothèses auditives, tel que représenté sur la figure 1, les sons 4 sont captés par un microphone situé sur un boîtier externe 3, qui comprend en outre un microprocesseur pour numériser et traiter les sons ainsi captés et qui est en général un support de type contour d'oreille pour le placer discrètement en arrière de celle-ci 8. Dans un autre mode de réalisation ledit boîtier externe 3 portant le microphone peut être placé dans l'oreille 8 et avoir alors une forme adaptée à celle du conduit auditif 8₁.

Ce boîtier externe 3 adresse soit par un petit câble de liaison 3₁, soit par un jeu d'antennes, les signaux ainsi traités à un dispositif dit également externe 2 qui comprend au moins une bobine : celle-ci peut en effet faire office d'antenne comme indiqué précédemment ; on la considérera ainsi dans toute la suite de la description à titre d'exemple de réalisation, mais dans d'autres cas, tout en restant dans le cadre de la présente invention, l'antenne pourrait être indépendante de la bobine, et même séparée du dispositif externe 2.

Celui-ci est maintenu contre la peau 9 en face d'un dispositif implanté 1 grâce à un jeu d'au moins deux aimants disposés et solidaires chacun d'un des dispositifs, le dispositif implanté 1 étant maintenu quant à lui contre l'os 7 de la boîte crânienne.

Ledit implant 1 reçoit alors les signaux d'informations du dispositif extérieur 2 grâce à sa propre bobine/antenne : comme indiqué ci-dessus il pourrait recevoir ces signaux depuis un émetteur/antenne distinct de ce dispositif extérieur, et même directement depuis un microphone, qui pourrait être autonome et disposant d'une antenne, et/ou qui pourrait être lui-même implantable et capterait les données de base que sont les sons en provenance de l'extérieur du corps. L'electronique 15 de l'implant 1 répartit ces signaux vers et sur les électrodes 10 placées dans la cochlée 5 : les terminaisons du nerf auditif 6 en contact avec ces électrodes transmettent les impulsions électriques jusqu'au cerveau qui les interprète comme des sons.

Selon une réalisation particulière de l'invention, le dispositif externe 2 peut comporter, en plus de la bobine/antenne et de l'aimant, d'autres éléments contenus traditionnellement dans le boîtier externe 3 tels que microprocesseur, pile ou batterie, microphone,... de sorte que le boîtier 3 n'est plus nécessaire. Dans cette configuration le patient ne porte en externe plus que le seul dispositif 2 fixé simplement sur la peau par aimantation avec le dispositif implanté.

Les figures 2A et 2B représentent un exemple de boîtier implantable 11, suivant une configuration telle que celle connue à ce jour : il contient l'électronique 15 qui soit envoie les impulsions électriques aux électrodes pour stimuler le nerf auditif, soit, dans d'autres applications, recueille les données en provenance des électrodes pour les transmettre vers le boîtier externe 2 : ces boîtiers implants connus sont disposés à ce jour à côté d'un support/enveloppe 14 réalisé en général en silicone et qui contient la bobine/antenne 12 et l'aimant 13 de maintien et de centrage par attraction de l'aimant situé dans le dispositif extérieur 2.

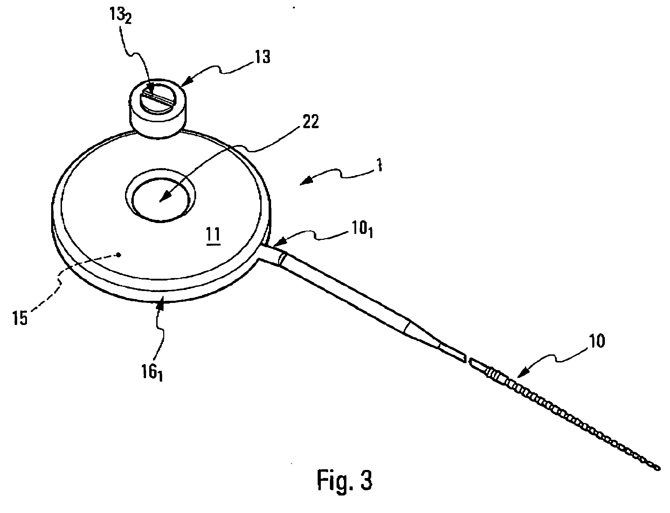

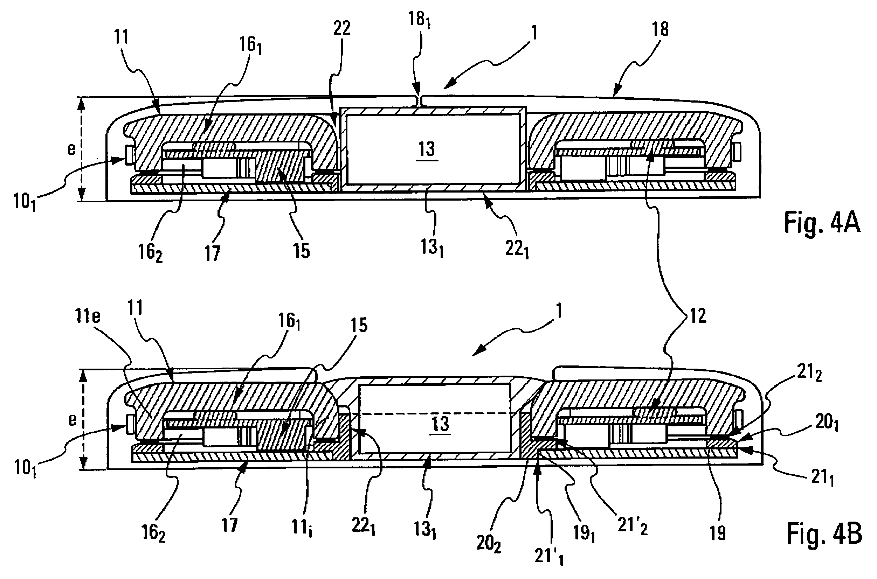

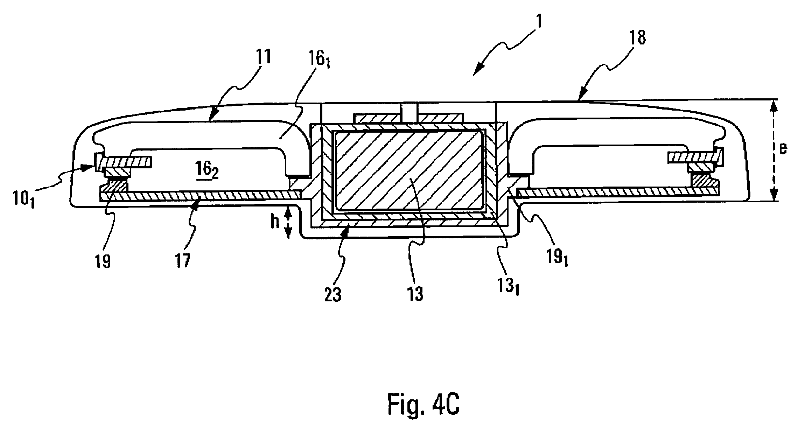

Suivant la présente invention tel que représenté dans les figures 3 et 4, le boîtier 11 qui contient au moins les moyens électroniques 15 et ladite bobine/antenne 12 comporte une couronne 16₁ creuse, formant une gorge annulaire en forme de U que l'on distingue bien sur les figures en coupe 4A, 4B et 4C, réalisée en matériau biocompatible rigide et perméable aux ondes électromagnétiques, tel qu'en céramique.

L'ouverture de la partie creuse 16₂ de ladite couronne 16 est fermée de manière étanche par un fond 17 en matériau biocompatible tel que du titane.

Le dispositif implantable 1 comporte également un aimant 13 de dimensions compatibles avec le logement central 22 formé par la paroi dite intérieure de la couronne 16₁ et qui est en fait l'aile 11ᵢ de la forme en U de celle-ci orientée vers le centre du boîtier 11, lequel logement étant ouvert au moins d'un côté car traversant au milieu du boîtier 11, et dans lequel l'aimant est introduit, placé et éventuellement fixé tout en étant extractible tel que représenté sur la figure 3.

Cet aimant 13 est encapsulé dans une enveloppe 13₁ en matériau biocompatible rigide tel qu'en titane qui, pour permettre la fixation de l'aimant dans le logement central 22 d'une manière réversible et extractible, est soit vissée dans le cylindre formé par ce logement central 22 et doté d'un pas de vis- compatible (comme représenté sur la figure 4B), soit clipsée dans le fond 17 du boîtier 11, quand ledit fond 17 ferme non seulement l'ouverture de la partie creuse 16₂ de la couronne 16₁ mais également celle 22₁ du côté correspondant du logement 22. Dans ce dernier cas le logement 22 est ouvert des deux côtés.

Dans le mode de réalisation représenté sur la figure 3, l'aimant 13 comporte une fente 13₂ permettant de le visser et dévisser dans un filetage réalisé soit dans le logement 22 soit dans un insert en titane ostéointégrable et préalablement fixé dans l'os du corps considéré.

Dans un mode particulier de réalisation représenté sur la figure 4C l'aimant 13 peut dépasser du fond 17, à l'opposé de la face de l'implant qui est destinée a être orientée vers l'extérieur du corps vivant : la partie de l'aimant 13, de son enveloppe 13₁ et du réceptacle 23 de celle-ci, lequel réceptacle est solidaire de la couronne 16₁ creuse du boîtier 11, et dépasse ainsi d'une hauteur "h" du fond 17, et est destinée à être logée dans une cavité fraisée préalablement dans l'os, tel que l'os temporal de la boîte crânienne pour un implant cochléaire, dudit corps vivant à l'endroit voulu. Cette disposition permet d'une part de réduire l'épaisseur "e" du boîtier 11, car non lié alors à celle de l'aimant 13, et le diamètre de celui-ci, et donc la surface de l'ensemble du dispositif implantable, et d'autre part d'augmenter l'épaisseur de l'aimant implanté ce qui favorise l'attractivité de l'aimant extérieur et donc une meilleure tenue du dispositif externe 2. Cette force d'aimantation plus importante est appréciable dans la configuration décrite plus haut, où le dispositif externe 2 comporte plus de composants, tels que la pile et le microprocesseur, et est, de ce fait, plus lourd. Ce dispositif assure également un meilleur maintien de l'implant du fait de son emboîtement mécanique par rapport à l'os, et donc réduit l'effet de traction généré par le poids de l'ensemble sur la peau.

Selon un mode de réalisation représenté sur la figure 4A, l'enveloppe en matériau biocompatible encapsulant l'aimant 13 est glissée et maintenue dans le logement 22 par une enveloppe externe 18 en matériau biocompatible souple tel qu'en silicone et perméable aux ondes électromagnétiques, qui entoure tout le dispositif et qui comporte une fente 18₁ apte à permettre le passage dudit aimant 13. Cette enveloppe externe 18 n'est pas représentée sur la figure 3 pour bien faire apparaître la forme en couronne 16₁ du boîtier 11 et le logement central 22 apte à recevoir l'aimant 13.

Pour assurer une bonne fixation étanche entre le fond 17 de préférence en titane et le boîtier 16 en céramique, et afin de limiter le risque de détérioration de celui-ci, ce fond 17 en titane est soudé 21₁ de préférence au laser au moins en périphérie sur une couronne 19 également de préférence en titane, brasée, à l'or de préférence, elle-même 21₂ préalablement sur la paroi dite extérieure formant la périphérie radiale du boîtier 11 de la couronne creuse 16₁, (correspondant à l'aile 11ₑ de la forme en U de celle-ci, tournée vers l'extérieur du boîtier 11) : ladite soudure 21₁ est décalée vers l'extérieur et radialement dans le plan de la couronne 19 grâce à une lèvre 20₁ de celle-ci, par rapport à la brasure 21₂ qui étant ainsi éloignée du point de chauffe de la soudure 21₁ n'est pas détériorée.

Un assemblage similaire est réalisé pour la soudure du fond 17 en périphérie intérieure autour du logement 22, tel que représenté sur la figure 4B : la soudure 21'₁ du fond 17 en titane sur une couronne 19₁, pouvant faire partie du réceptacle 23 de l'aimant 13 comme représenté sur la figure 4c, est décalée vers l'extérieur, et axialement perpendiculairement au plan du fond 17, grâce à une lèvre 20₂ correspondant à une première branche de ladite couronne 19₁ ; une deuxième branche de celle-ci perpendiculaire à la première est brasée 21'₂ sur la paroi dite intérieure 11ᵢ de la couronne creuse 16₁. Les deux dites branches de cette couronne 19₁ forment ainsi un L comme représenté sur la figure 4_{A}, mais cette couronne peut être aussi en forme de T comme représenté sur la figure 4_{B}, sa troisième branche, perpendiculaire également au plan du fond 17, remontant dans le logement 22 pour permettre le vissage de l'aimant 13 grâce a des pas de vis 22₁ respectivement compatibles.

L'invention est définie dans le jeu de revendications suivant.

## Revendications

1. Dispositif implantable (1) dans un corps vivant, sous-cutané et apte à émettre et/ou recevoir des données en direction ou en provenance de l'extérieur du corps et à recevoir de l'énergie par couplage électromagnétique avec une bobine d'un dispositif (2) externe, ledit dispositif implantable comportant pour cela au moins une bobine (12), un boîtier (11) étanche contenant des moyens électroniques (15) au moins de stimulation et/ou de recueil, un aimant (13) apte à maintenir et à centrer la bobine dudit dispositif externe (2) avec ladite au moins une bobine (12) du dispositif implantable,
**caractérisé en ce que :**
- ledit boîtier (11) comporte :
• une couronne (16₁) creuse en matériau biocompatible et perméable aux ondes électromagnétiques, comportant :
▪ une paroi extérieure (11ₑ) formant la périphérie radiale du boîtier (11), et
▪ une paroi intérieure (11ᵢ) orientée vers le centre du boitier étanche (11) et formant un logement central (22) au milieu du boitier étanche (11),
▪ la paroi extérieure et la paroi intérieure (11ᵢ) formant l'ouverture d'une gorge annulaire en forme de U.
• un fonds (17) en matériau biocompatible fermant de manière étanche l'ouverture de la partie creuse (16₂) de la couronne (16₁), et
• la partie creuse (16₂) de la couronne (16₁) contenant au moins les moyens électroniques (15) et ladite au moins une bobine (12).
- ledit aimant (13) est de dimensions compatibles avec le logement central (22) formé par la paroi intérieure (11i) de la couronne (161) et est placé dans ledit logement central (22) au milieu du boîtier étanche (11) de manière réversible et extractible.

2. Dispositif selon la revendication 1, dans lequel l'aimant (13) est encapsulé dans une enveloppe (13₁) en matériau biocompatible qui est vissée dans le cylindre formé par le logement central (22) et doté d'un pas de vis compatible.

3. Dispositif selon la revendication 1, dans lequel l'aimant (13) est encapsulé dans une enveloppe (13₁) en matériau biocompatible qui est clipsée dans le fond (17) du boîtier (11).

4. Dispositif selon la revendication 1, dans lequel l'aimant (13) est encapsulé dans une enveloppe (13₁) en matériau biocompatible qui est glissée et maintenue dans le logement (22) par une enveloppe externe (18) en matériau biocompatible et perméable aux ondes électromagnétiques, qui entoure tout le dispositif et qui comporte une fente apte à permettre le passage dudit aimant (13).

5. Dispositif selon l'une quelconque des revendications 1 à 4, dans lequel le matériau de la couronne (16₁) biocompatible et perméable aux ondes électromagnétiques est de la céramique.

6. Dispositif selon l'une quelconque des revendications 1 à 5, dans lequel le matériau du fond (162) est du titane.

7. Dispositif selon l'une quelconque des revendications 5 et 6, dans lequel le fond (17) en titane est soudé (21₁) au moins en périphérie extérieure sur une couronne (19) brasée elle-même (21₂) préalablement sur la paroi dite extérieure formant la périphérie radiale de la couronne creuse (16₁), ladite soudure (21₁) étant décalée grâce à des lèvres (20) par rapport à la brasure (21₂).

8. Dispositif selon l'une quelconque des revendications 1 à 7, lequel comprend au moins une électrode filaire (10) extérieure dont l'extrémité distale est apte à être logée contre ou dans tout organe du corps vivant vers lequel et/ou duquel lesdites données sont adressées et/ou recueillies par les moyens électroniques (15).

9. Dispositif selon la revendication 8, dans lequel ladite électrode filaire (10) est connectée par son extrémité proximale (10₁) à la périphérie radiale de la couronne creuse (16₁) du boitier (11).

10. Dispositif selon une des revendications 8 ou 9, dans lequel ladite électrode filaire (10) est une électrode cochléaire.

11. Dispositif selon l'une quelconque des revendications 1 à 10, dans lequel ledit aimant (13) dépasse du fond (17) du boîtier (11), à l'opposé de la face de l'implant destinée à être orientée vers l'extérieur du corps vivant.

## Patentansprüche

1. Subkutan in einen lebenden Körper implantierbare Vorrichtung (1), die zum Aussenden und/oder Empfangen von Daten in Richtung oder aus der Umgebung des Körpers und zum Empfangen von Energie durch elektromagnetische Kopplung mit einer Spule einer externen Vorrichtung (2) geeignet ist, wobei die implantierbare Vorrichtung hierzu mindestens ein
eine Spule (12),
ein dichtes, wenigstens elektronische Stimulations- und/oder Sammelmittel (15) und
einen zum Zentrieren der Spule der externen Vorrichtung (2) mit der mindestens einen Spule (12) der implantierbaren Vorrichtung geeigneten Magneten (13)
enthaltendes Gehäuse (11) aufweist,
**dadurch gekennzeichnet, dass** das Gehäuse (11) aufweist:
eine hohle Krone (16₁) aus biokompatiblem und für elektromagnetische Wellen durchlässigem Material, aufweisend:
eine äußere Wand (11ₑ), die den radialen Umfang des Gehäuses (11) bildet, und
eine innere Wand (11ᵢ), die zum Zentrum des dichten Gehäuses (11) orientiert ist und eine zentrale Aufnahme (22) in der Mitte des dichten Gehäuses (11) bildet,
wobei die äußere Wand und die innere Wand (11ᵢ) eine Öffnung einer ringförmigen Ausnehmung in Form eines U bilden,
einen Boden (17) aus biokompatiblem Material, der die Öffnung des hohlen Teils (16₂) der Krone (16₁) dicht abschließt, und
wobei der hohle Teil (16₂) der Krone (16₁) zumindest die elektronischen Mittel (15) und die mindestens eine Spule (12) enthält,
wobei der Magnet (13) mit der von der inneren Wand (11ᵢ) der Krone (16₁) gebildeten zentralen Aufnahme (22) kompatible Abmessungen hat und in der zentralen Aufnahme (22) in der Mitte des dichten Gehäuses (11) lösbar und entnehmbar angeordnet ist.

2. Implantierbare Vorrichtung nach Anspruch 1, bei der der Magnet (13) von einer Hülle (13ₗ) aus biokompatiblem Material umschlossen ist, die in den von der zentralen Aufnahme (22) gebildeten und mit einem kompatiblen Gewinde ausgestatteten Zylinder geschraubt ist.

3. Implantierbare Vorrichtung nach Anspruch 1, bei der der Magnet (13) von einer Hülle (13ₗ) aus biokompatiblem Material umschlossen ist, die in den Boden (17) des Gehäuses (11) geklipst ist.

4. Implantierbare Vorrichtung nach Anspruch 1, bei der der Magnet (13) von einer Hülle (13ₗ) aus biokompatiblem Material umschlossen ist, die durch eine äußere Hülle (18) aus biokompatiblem und für elektromagnetische Wellen durchlässigem Material in die Aufnahme (22) geschoben und in dieser gehalten ist, wobei die äußere Hülle die gesamte Vorrichtung umgibt und einen Schlitz aufweist, der geeignet ist, den Magneten (13) hindurch zu lassen.

5. Implantierbare Vorrichtung nach einem der Ansprüche 1 bis 4, bei der das biokompatible und für elektromagnetische Wellen durchlässige Material der Krone (16₁) Keramik ist.

6. Implantierbare Vorrichtung nach einem der Ansprüche 1 bis 5, bei der das Material des Bodens (16₂) Titan ist.

7. Implantierbare Vorrichtung nach einem der Ansprüche 5 und 6, bei der der Boden (17) aus Titan zumindest an seinem äußerem Umfang an eine Krone (19) geschweißt ist (21₁), die ihrerseits zuvor an die äußere Wand gelötet ist (21₂), die den radialen Umfang der hohlen Krone (16₁) bildet, wobei die Verschweißung durch Lippen (20) bezüglich der Lötung (21₂) versetzt ist.

8. Implantierbare Vorrichtung nach einem der Ansprüche 1 bis 7, die mindestens eine äußere Drahtelektrode (10) aufweist, deren distales Ende dazu geeignet ist, an oder in einem Organ des lebenden Körpers angeordnet zu werden, an den oder von dem die Daten adressiert und/oder durch die elektronischen Mittel (15) gesammelt sind.

9. Implantierbare Vorrichtung nach Anspruch 8, bei der die Drahtelektrode (10) an ihrem proximalen Ende (10₁) mit dem radialen Umfang der hohlen Krone (16₁) des Gehäuses (11) verbunden ist.

10. Implantierbare Vorrichtung nach einem der Ansprüche 8 oder 9, bei der die Drahtelektrode (10) eine Cochlea-Elektrode ist.

11. Implantierbare Vorrichtung nach einem der Ansprüche 1 bis 10, bei der Magnet (13) auf der Seite aus dem Boden (17) des Gehäuses (11) hinausragt, die der Seite gegenüberliegt, die dazu bestimmt ist zum äußeren des lebenden Körpers orientiert zu sein.

## Claims

1. A device (1) implantable in a living body, subcutaneously, and adapted to emit and/or receive data to or from the outside of the body and to receive energy by electromagnetic coupling with a coil of an external device (2), said implantable device including for that purpose at least one coil (12), a tight case (11) containing electronic means (15) at least for stimulation and/or collection, a magnet (13) adapted to hold and centre the coil of said external device (2) with said at least one coil (12) of the implantable device, **characterized in that**:
- said case (11) includes :
• a hollow crown (16₁) made of a biocompatible and electromagnetic wave permeable material, including :
▪ an external wall (11e) forming the radial periphery of the case (11), and
▪ an internal wall (11ᵢ) oriented towards the centre of the tight case (11) and forming a central housing (22) in the middle of the tight case (11),
▪ the external wall and the internal wall (11ᵢ) forming the opening of a U-shaped annular groove,
• a bottom (17) made of a biocompatible material tightly closing the opening of the hollow part (16₂) of the crown (16₁), and
• the hollow part (16₂) of the crown (16₁) containing at least the electronic means (15) and said at least one coil (12),
- said magnet (13) has a size compatible with the central housing (22) formed by the internal wall (11ᵢ) of the crown (16₁) and is placed in said central housing (22) in the middle of the tight case (11) in a reversible and extractible manner.

2. The device according to claim 1, wherein the magnet (13) is encapsulated in an envelope (13₁) made of a biocompatible material, that is screwed into the cylinder formed by the central housing (22) and provided with a compatible screw pitch.

3. The device according to claim 1, wherein the magnet (13) is encapsulated in an envelope (13₁) made of a biocompatible material, that is clipsed into the bottom (17) of the case (11).

4. The device according to claim 1, wherein the magnet (13) is encapsulated in an envelope (13₁) made of a biocompatible material, that is slid into and held in the housing (22) by an external envelope (18) made of a biocompatible and electromagnetic wave permeable material, that surrounds the whole device and that includes a slot adapted to let said magnet (13) through.

5. The device according to any one of claims 1 to 4, wherein the biocompatible and electromagnetic wave permeable material of the crown (16₁) is ceramic.

6. The device according to any one of claims 1 to 5, wherein the material of the bottom (16₂) is titanium.

7. The device according to any one of claims 5 and 6, wherein the titanium bottom (17) is welded (21₁) at least at its external periphery to a crown (19), itself previously soldered (21₂) to the so-called external wall forming the radial periphery of the hollow crown (16₁), said weld (21₁) being offset thanks to lips (20) with respect to the solder (21₂).

8. The device according to any one of claims 1 to 7, which comprises at least one external wire electrode (10), whose distal end is adapted to be received against or within any organ of the living body to and/or from which said data are addressed and/or collected by the electronic means (15).

9. The device according to claim 8, wherein said wire electrode (10) is connected by its proximal end (10₁) to the radial periphery to the hollow crown (16₁) of the case (11).

10. The device according to one of claims 8 or 9, wherein said wire electrode (10) is a cochlear electrode.

11. The device according to any one of claims 1 to 10, wherein said magnet (13) protrudes from the bottom (17) of the case (11), opposite to the face of the implant intended to be oriented towards the outside of the living body.
